# EUROPEAN PATENT APPLICATION

(11) **EP 4 245 309 A1**
(43) Date of publication of application: **20.09.2023**
(21) Application number: 21891278.0
(22) Date of filing: 16.11.2021
(51) Int. Cl.: A61K 35/745, A61K 31/702, A61P 31/04

(54) **COMPOSITION CONTAINING BIFIDOBACTERIUM LACTIS AND HUMAN MILK OLIGOSACCHARIDES AND APPLICATION THEREOF**

(30) Priority: 16.11.2020 CN 202011279614
(71) Applicant: Inner Mongolia Yili Industrial Group Co., Ltd., Inner Mongolia 010110 (CN); Inner Mongolia Dairy Tech Res Institute Co Ltd, Hohhot, Inner Mongolia 010110 (CN)
(72) Inventor: WEISS, Gisela Adrienne, Hohhot City, Inner Mongolia 010110 (CN); VAN LOO-BOUWMAN, Carolien Annika, Hohhot City, Inner Mongolia 010110 (CN); SMIT, Gerrit, Hohhot City, Inner Mongolia 010110 (CN); WANG, Wendan, Hohhot City, Inner Mongolia 010110 (CN); SZETO, Ignatius Man-Yau, Hohhot City, Inner Mongolia 010110 (CN); GU, Fangjie, Hohhot City, Inner Mongolia 010110 (CN); LIU, Biao, Hohhot City, Inner Mongolia 010110 (CN)
(74) Representative: Dehns
(86) International application number: PCT/CN2021/130978
(87) International publication number: WO 2022/100758

(57) **Abstract**

The present disclosure provides a composition comprising *Bifidobacterium lactis* and human milk oligosaccharides (HMOs) and use thereof. The composition is capable of effectively enhancing resistance of an organism to *Staphylococcus aureus* infection, improving innate immunity of an organism, and/or anti-aging, and can be added to various health foods and healthcare foods.

## Description

### Technical Field

The present disclosure mainly relates to a probiotic-prebiotic composition for enhancing resistance of an organism to *Staphylococcus aureus* infection and the use thereof, more particularly to a composition comprising *Bifidobacterium lactis* HN019 and human milk oligosaccharides (HMOs), which can effectively enhance resistance of an organism to *Staphylococcus aureus* infection, and can be added to various health foods and healthcare foods.

### Background

Over the last millennium, it has been documented in the medical literature that infants who were not breastfed suffered higher morbidity and mortality. Breast milk not only provides necessary nutrition for infants, but contains active ingredients that provide support for the development of infants' intestinal tract and immunity. Compared with formula-fed infants, breastfed infants have a higher relative abundance of beneficial bacteria in their intestinal flora, especially *Bifidobacteria* and lactic acid bacteria.

Breast milk establishes healthy intestinal flora for newborns by delivery of microbiota and active ingredients such as HMOs and cytokines in breast milk. An infant takes up 10⁷ to 10⁸ bacteria every day through breast milk, including lactic acid bacteria and *Bifidobacteria.* These bacteria are transferred directly to the infant through breast milk, and some of them can colonize the infant's intestinal tract, promoting the establishment of intestinal flora in the early stage of life. The establishment of intestinal flora in an infant has both short-term and life-long effects on the development of the intestinal tract as well as on the immune system and the health.

Human milk oligosaccharides (HMOs) are the third most abundant substance in human milk after lactose and fat. The total content varies among different stages of lactation, and is approximately 12 to 14 g/L in mature milk, and approximately 20 to 24 g/L in colostrum. In the structure of each human milk oligosaccharide, a lactose is present at the reducing end, and most HMOs have a polylactosamine skeleton, with fucose and/or sialic acid at the end of the chain. Human milk oligosaccharides are mainly composed of three types: (1) fucosyl-based oligosaccharides, represented by 2'-fucosyl oligosaccharide and 3'-fucosyl oligosaccharide; (2) sialyl-based oligosaccharides, represented by 3'-sialyllactose and 6'-sialyllactose; (3) oligosaccharides formed by a core sugar chain structure without a fucosyl or sialyl group, represented by lacto-N-tetraose and lacto-N-neotetraose.

The presence and content of HMOs vary among individuals and are associated with the Lewis secretor phenotype of the mother. Since the raw material of infant formula powder is generally cow's milk which usually contains little or no such oligosaccharides, HMOs are a gap that must be overcome to make an infant formula closer to human milk.

In the 1990s, 2'-fucosyllactose (2'-FL), an HMO present in most breast milk, was found to be effective in attenuating the toxicity of stable toxins in *Escherichia. coli.* In 2003, this oligosaccharide was reported to inhibit the attachment and infection by *Campylobacter jejuni.* Subsequently, the three major functions of HMOs were gradually discovered and reported: (1) inhibiting attachment and infection by certain pathogenic bacteria; (2) serving as a prebiotic to promote the growth of bacteria in the intestinal symbiotic system; and (3) directly retarding the inflammatory response of mucous membranes to noxious stimuli. The first clinical trial with 2'-FL demonstrated that its addition in a low-calorie formula was not only safe but also allowed formula-fed infants to grow at a rate comparable to that of breastfed infants. 2'-FL has also been used as a nutritional supplement for adults to relieve irritable bowel syndrome or inflammatory bowel diseases, or as a prebiotic to maintain the balance of intestinal flora.

Intestinal flora is an important component of the human intestinal micro-ecosystem and plays an important role in human health. For example, it provides essential nutrients, produces vitamin K, assists the digestive process, and promotes angiogenesis and intestinal nerve functioning. Both prebiotics and probiotics are considered as microecological management tools to improve the health of an organism, and can modify, regulate, and reorganize the existing intestinal flora.

At present, in the fields of infant formula powder, supplementary food and nutritional supplements, there is a need for a solution to relieve an intestinal discomfort of infants and enhance their ability to resist infection by pathogenic bacteria such as *Staphylococcus aureus.* Furthermore, for children over 3 years old, adolescents and adults, there is also a need for a solution to relieve an intestinal discomfort and enhance their ability to resist infection by pathogenic bacteria such as *Staphylococcus aureus.* As a model organism, *Caenorhabditis elegans* (*C. elegans*) has a great prospect of application in preclinical research and evaluation. It has a short life cycle (21 days), is highly replicable and reproducible, easy to operate, transparent, and easy to culture. Its genome has been fully sequenced, and a quarter of its genes are homologous to those in the human genome. Genetically mutated *C*. *elegans* generated by gene editing may serve as an experimental means for genetic analysis. Currently, *C*. *elegans* is not considered as an animal in European legislation. It is widely used in *in vitro* assays, for example in transcriptomics, proteomics, metabolomics and the like. As a model organism, it is often used as the first step in evaluation of a raw material. Prior to design of functional raw materials, *in vitro* enzymatic or cell assays, mouse modeling and clinical trials, *C*. *elegans* is often used as a high-throughput means to screen test raw materials for certain characteristics.

*Staphylococcus aureus* (*S. aureus*) is a Gram-positive bacterium belonging to the genus *Staphylococcus* and is a common food-borne pathogenic microorganism. It widely exists in the natural environment, and under certain conditions can produce enterotoxins and cause food poisoning. It has caused numerous incidents of food poisoning due to food-borne microbes. Once infected by *Staphylococcus aureus,* people may have common food poisoning symptoms such as nausea, vomiting, and dizziness, and may even have symptoms such as enteritis, pneumonia, skin infection, wound infection and ulceration, meningitis, and the like.

### Summary of the invention

An objective of the present disclosure is to provide a composition for enhancing resistance of an organism to *Staphylococcus aureus* infection.

The inventors have discovered in research that a combination of *Bifidobacterium lactis* and HMOs has a synergistic effect in enhancing resistance of an organism to *Staphylococcus aureus* infection.

Accordingly, in one aspect, the present disclosure provides a composition comprising *Bifidobacterium lactis* and human milk oligosaccharides (HMOs).

According to embodiments of the present disclosure, in the composition of the present disclosure, the *Bifidobacterium lactis* includes the bacterial strain *Bifidobacterium lactis* HN019.

The *Bifidobacterium lactis* HN019 strain is a commercial strain available from DuPont Danisco Co.

According to an embodiment of the present disclosure, in the composition of the present disclosure, the HMOs include one or more of 2'-fucosyllactose, 3'-fucosyllactose, lactose-N-tetraose, 3'-sialyllactose, and 6'-sialyllactose.

2'-fucosyllactose (2'-FL) is a trisaccharide structure formed by fucose and lactose, and is a representative of fucosyl-based oligosaccharides. Its commercial products are generally prepared by microbial fermentation and have the same structure as the oligosaccharide found in human milk.

3-fucosyllactose (3-FL) is a trisaccharide structure formed by fucose and lactose, a mutual isomer of 2'-fucosyllactose, and is a representative of fucosyl-based oligosaccharides. It is prepared by microbial fermentation and has the same structure as the oligosaccharide found in human milk.

Lacto-N-tetraose (LNT) is a hexasaccharide structure formed by lactose and a tetraose. It is prepared by microbial fermentation and is a representative of oligosaccharides having a core sugar chain as a basic structure and having no fucosyl or sialyl, and also has the same structure as the oligosaccharide found in human milk.

3'-sialyllactose (3'-SL) is a trisaccharide structure formed by sialic acid and lactose, a mutual isomer of 6'-sialyllactose, and is a representative of sialyl-based oligosaccharides. It is prepared by microbial fermentation and has the same structure as the oligosaccharide found in human milk.

6'-sialyllactose (6'-SL) is a trisaccharide structure formed by sialic acid and lactose, a mutual isomer of 3'-sialyllactose, and is a representative of sialyl-based oligosaccharides. It is prepared by microbial fermentation and has the same structure as the oligosaccharide found in human milk.

According to embodiments of the present disclosure, in the composition of the present disclosure, the content of 2'-fucosyllactose in the HMOs is 0 to 73%, preferably 0 to 63%, further preferably 0 to 58%, even further preferably 2 to 55%, for example, 3 to 8%, 25 to 35%, or 51 to 56%, and more particularly 5%, 30%, or 53%. In the present disclosure, unless specifically indicated, the content of each HMO is based on a total weight of 100% of the sum of 2'-fucosyllactose, 3'-fucosyllactose, lacto-N-tetraose, 3'-sialyllactose, and 6'-sialyllactose.

According to embodiments of the present disclosure, in the composition of the present disclosure, the content of 3'-fucosyllactose in the HMOs is 0 to 61%, preferably 11 to 51%, further preferably 16 to 46%, even further preferably 20 to 44%, for example, 18 to 23%, 25 to 35%, or 40 to 45%, and more particularly 21%, 30%, or 41%.

According to embodiments of the present disclosure, in the composition of the present disclosure, the content of lacto-N-tetraose in the HMOs is 0 to 52%, preferably 6 to 42%, further preferably 11 to 37%, even further preferably 13 to 35%, for example, 13 to 18%, 22 to 27%, or 30 to 35%, and more particularly 16%, 25%, or 32%.

According to embodiments of the present disclosure, in the composition of the present disclosure, the content of 3'-sialyllactose in the HMOs is 0 to 42%, preferably 0 to 32%, further preferably 0 to 27%, even further preferably 2 to 25%, for example, 2 to 7%, 10 to 15%, or 20 to 25%, and more particularly 5%, 12%, or 22%.

According to embodiments of the present disclosure, in the composition of the present disclosure, the content of 6'-sialyllactose in the HMOs is 0 to 25%, preferably 0 to 15%, further preferably 0 to 10%, even further preferably 0 to 8%, for example, 3 to 8%, and more particularly 3% or 5%.

According to embodiments of the present disclosure, in the composition of the present disclosure, the HMOs are a combination of at least two, at least three, or at least four of 2'-fucosyllactose (2'-FL), 3-fucosyllactose (3-FL), lactose-N-tetraose (LNT), 3'-sialyllactose (3'-SL), and 6'-sialyllactose (6'-SL).

According to embodiments of the present disclosure, in the composition of the present disclosure, the HMOs include at least three or four of 2'-fucosyllactose, 3'-fucosyllactose, lactose-N-tetraose, 3'-sialyllactose, and 6'-sialyllactose in a weight ratio of (0 to 73%) : (0 to 61%) : (0 to 52%) : (0 to 42%) : (0 to 25%).

According to embodiments of the present disclosure, in the composition of the present disclosure, the HMOs include at least three or four of 2'-fucosyllactose, 3'-fucosyllactose, lactose-N-tetraose, 3'-sialyllactose, and 6'-sialyllactose in a weight ratio of (0 to 63%) : (11 to 51%) : (6 to 42%) : (0 to 32%) : (0 to 15%).

According to embodiments of the present disclosure, in the composition of the present disclosure, the HMOs include at least three or four of 2'-fucosyllactose, 3'-fucosyllactose, lactose-N-tetraose, 3'-sialyllactose, and 6'-sialyllactose in a weight ratio of (0 to 58%) : (16 to 46%) : (11 to 37%) : (0 to 27%) : (0 to 10%).

According to embodiments of the present disclosure, in the composition of the present disclosure, the HMOs include 2'-fucosyllactose, 3'-fucosyllactose, lactose-N-tetraose, 3'-sialyllactose, and 6'-sialyllactose in a weight ratio of (0 to 55%) : (20 to 44%) : (13 to 35%) : (2 to 25%) : (0 to 8%).

According to embodiments of the present disclosure, in the composition of the present disclosure, the HMOs include 2'-fucosyllactose, 3'-fucosyllactose, lactose-N-tetraose, 3'-sialyllactose, and 6'-sialyllactose in a weight ratio of (0 to 53%) : (21 to 41%) : (16 to 32%) : (5 to 22%) : (0 to 5%), preferably (5 to 53%) : (21 to 41%) : (16 to 32%) : (5 to 22%) : (0 to 5%), and more preferably (5 to 53%) : (21 to 41%) : (16 to 32%) : (5 to 22%) : (3 to 5%).

According to embodiments of the present disclosure, in the composition of the present disclosure, the HMOs include 2'-fucosyllactose, 3'-fucosyllactose, lactose-N-tetraose, 3'-sialyllactose, and 6'-sialyllactose in a weight ratio of (30 to 53%) : (21 to 30%) : (16 to 25%) : (5 to 12%) : (3 to 5%).

According to embodiments of the present disclosure, in the composition of the present disclosure, the HMOs include 2'-fucosyllactose, 3'-fucosyllactose, lactose-N-tetraose, 3'-sialyllactose, and 6'-sialyllactose in a weight ratio of (33 to 73%) : (1 to 41%) : (0 to 36%) : (0 to 25%) : (0 to 25%), preferably (43 to 63%) : (11 to 31%) : (6 to 26%) : (0 to 15%) : (0 to 15%), more preferably (48 to 58%) : (16 to 26%) : (11 to 21%) : (0 to 10%) : (0 to 10%), and most preferably (51 to 56%) : (18 to 23%) : (13 to 18%) : (2 to 7%) : (3 to 8%).

According to an embodiment of the present disclosure, in the composition of the present disclosure, the ratio of the *Bifidobacterium lactis* to the HMOs is (1× 10³ to 1×10¹² CFU) : (0.1 to 10 g), preferably (1×10⁶ to 1×10¹⁰ CFU) : (1 to 10 g). According to certain embodiments of the present disclosure, the ratio of the *Bifidobacterium lactis* to the HMOs is 1×10⁸ CFU : 0.08 to 0.3 g. The ratio of the *Bifidobacterium lactis* to the HMOs is based on their amounts in the same composition.

In a further aspect, the present disclosure provides the use of the composition in the manufacture of a food product or medicament for effectively enhancing resistance of an organism to *Staphylococcus aureus* infection, improving innate immunity of an organism, and/or anti-aging.

According to an embodiment of the present disclosure, in the use of the composition of the present disclosure, the organism includes an animal and human.

According to an embodiment of the present disclosure, in the use of the composition of the present disclosure, the enhancing resistance of an organism to *Staphylococcus aureus* infection includes: enhancing an individual's ability to prevent *S. aureus* infection, reducing the ability of *S. aureus* to infect an individual, and/or alleviating symptoms caused by *S. aureus* infection such as food poisoning (including nausea, vomiting and dizziness), enteritis, pneumonia, skin infection, wound ulceration, or meningitis.

According to an embodiment of the present disclosure, the composition of the present disclosure may be used to prepare various health foods, healthcare foods, and medicaments. Particularly, the food may be a liquid beverage, a solid beverage, an oral liquid, a diary product, a tablet, or a capsule. For example, it may be used as a supplement in infant food (including infant formula powder, supplementary food, nutritional supplement) and nutritional supplement or food for children at the age of 3 or above, adolescents and adults (e.g., milk powder), which may find broad applications. The medicament may be an oral preparation, or may be an external formulation, such as an ointment for application.

In another aspect, the present disclosure provides a method for enhancing resistance of an organism to *Staphylococcus aureus* infection, improving innate immunity of an organism, and/or anti-aging, which comprises: administering to the organism an effective amount of the composition according to the present disclosure. Here, the enhancing resistance of an organism to *Staphylococcus aureus* infection includes: enhancing an individual's ability to prevent *S. aureus* infection, reducing the ability of *S. aureus* to infect an individual, and/or alleviating food poisoning, enteritis, pneumonia, skin infection, wound ulceration, and/or meningitis caused by *S. aureus* infection in an individual. In certain embodiments of the present disclosure, the composition is administered to the organism in the form of food, wherein the food is a liquid beverage, a solid beverage, an oral liquid, a diary product, a tablet, or a capsule, such as milk powder, particularly infant formula powder. Preferably, *Bifidobacterium lactis* in the food is used in an amount of 1×10³ to 1×10¹² CFU/day, and more preferably 1×10⁶ to 1×10¹¹ CFU/day. Preferably, the HMOs in the food are used in an amount of 0.015 to 15 g/day, further preferably 0.1 to 12 g/day, and more preferably 1.5 to 10 g/day.

The present disclosure further provides a food product comprising the composition according to the present disclosure.

According to an embodiment of the present disclosure, the *Bifidobacterium lactis* in the food product is used in an amount of 1×10³ to 1×10¹² CFU/day, and preferably 1×10⁶ to 1×10¹¹ CFU/day. In certain embodiments of the present disclosure, the *Bifidobacterium lactis* in the food product is used in an amount of 1×10⁸ CFU/day.

According to an embodiment of the present disclosure, the HMOs in the food product are used in an amount of 0.015 to 15 g/day, further preferably 0.1 to 12 g/day, and more preferably 1.5 to 10 g/day.

The food product or medicament according to the present disclosure comprising the composition can enhance resistance of an organism to *Staphylococcus aureus* infection by including the composition.

### Description of the Drawings

Fig. 1 shows the effect of a composition of 10 mg/mL of HMO mix A and *Bifidobacterium lactis* HN019 on the survival rate of *Caenorhabditis elegans* infected by *Staphylococcus aureus.* For each group indicated in the figure, the addition of intervening substance (probiotic and/or HMOs) in the infection stage is the same as that in the culture stage.
Fig. 2 shows the effect of a composition of 30 mg/mL of HMO mix A and *Bifidobacterium lactis* HN0 19 on the survival rate of *Caenorhabditis elegans* infected by *Staphylococcus aureus.* For each group indicated in the figure, the addition of intervening substance (probiotic and/or HMOs) in the infection stage is the same as that in the culture stage.
Fig. 3 shows the effect of a composition of HMO mix A and *Bifidobacterium lactis* HN019 on the survival rate of *Caenorhabditis elegans* on Day 3 after infected by *Staphylococcus aureus.* For each group indicated in the figure, the addition of intervening substance (probiotic and/or HMOs) in the infection stage is the same as that in the culture stage.
Fig. 4 shows the effect of a composition of HMO mix A and *Bifidobacterium lactis* HN019 on the survival rate of *Caenorhabditis elegans* on Day 5 after infected by *Staphylococcus aureus.* For each group indicated in the figure, the addition of intervening substance (probiotic and/or HMOs) in the infection stage is the same as that in the culture stage.
Fig. 5 is a picture of the results of the disc diffusion test of inhibition zone with gentamicin.
Fig. 6 is a picture of the results of the disc diffusion test of inhibition zone with *Bifidobacterium lactis* HN019.
Fig. 7 shows the different effects of the co-culture of HN019 (1×10⁸ CFU) and HMO mix A (10 mg/mL) in improving the survival rate of *C*. *elegans* before and/or during *S. aureus* infection.
Fig. 8 shows the effects of HMO mix A (8 mg/mL) and *Bifidobacterium lactis* HN019 (1×10⁸ CFU), alone and in combination, on the survival rate of *C*. *elegans* infected by *Staphylococcus aureus.*
Fig. 9 shows the effects of HMO mix G (8 mg/mL) and *Bifidobacterium lactis* HN019 (1×10⁸ CFU), alone and in combination, on the survival rate of *C*. *elegans* infected by *Staphylococcus aureus.*
Fig. 10 shows the effects of HMO mix H (8 mg/mL) and *Bifidobacterium lactis* HN019 (1 × 10⁸ CFU), alone and in combination, on the survival rate of *C*. *elegans* infected by *Staphylococcus aureus.*

In the figures: ^{∗} represents p<0.05, ^{∗∗} represents p<0.01, ^{∗∗∗} represents p<0.001, ^{∗∗∗∗} represents p<0.0001, NS represents no significant difference (not significant).

### Detailed Description of the invention

In order to provide a better understanding of the technical features, purposes and advantageous effects of the present disclosure, the technical solutions of the present disclosure are described in details hereinafter, which should not be construed as limitation to the implementable scope of the present disclosure.

**Example 1:** Effects of a composition of an HMO mix and *Bifidobacterium lactis* HN019 on the survival rate of *Caenorhabditis elegans* infected by *Staphylococcus*

### aureus

### 1. Experimental materials and methods

### 1.1 The tested HMO samples are shown in Table 1.

**Table 1**

| Name | English abbreviation | Source |
|---|---|---|
| 2'-fucosyllactose | 2'-FL | Jennewein |
| 3-fucosyllactose | 3-FL | Jennewein |
| Lacto-N-Tetraose | LNT | Jennewein |
| 3'-sialyllactose | 3'-SL | Jennewein |
| 6'-sialyllactose | 6'-SL | Jennewein |
| HMO mix A of 5 HMOs | HMO mix | Jennewein |

In HMO mix A, the five HMOs were in a ratio of: 53% 2'-FL, 21% 3-FL, 16% LNT, 5% 3'-SL, and 5% 6'-SL.

A solution of HMOs was prepared with distilled water and incubated in dishes containing a nematode growth medium at different final concentrations (1, 10, 30 mg/mL, respectively).

The probiotic to be tested was grown in an MRS medium supplemented with 1% cysteine, and incubated overnight at 37°C in an anaerobic environment. The cells were harvested and rinsed with physiological saline, the concentration of the bacteria was adjusted, and the bacteria were then inoculated on a plate containing a nematode growth medium at a final concentration of 1×10⁸ CFU.

### 1.2 Nematode infection model

Nematodes of the same age were obtained and cultured in a dish containing a nematode agar medium (NGM medium, a nematode medium containing *E. coli* OP50 as food), and a composition of an HMO mix at different dosages (8 mg/mL, 10 mg/mL, 30 mg/mL) and the probiotic (1×10⁸ CFU) was added for co-culturing (the total volume of liquid added to each nematode culture dish was 10 mL; for the test group having HMOs at a concentration of 10 mg/mL, the final amount of HMOs in each dish was 100 mg; for the test groups having HMOs at a concentration of 8 mg/mL and 30 mg/mL, the final amount of HMOs in each dish was 80 mg and 300 mg, respectively; thus, in each plate, the ratio of probiotic to HMOs was 1×10⁸ CFU : 80-300 mg). After the nematodes grew into an adult form, they were transferred to dishes inoculated with *Staphylococcus aureus* ATCC25923 at an amount of 10⁸-10⁹ CFU/mL to simulate the infection by *Staphylococcus aureus.* For each group, the addition of the intervening substance (probiotic and/or HMOs) in the infection stage was the same as that in the culture stage. Two controls were set to respectively create the condition without pathogenic bacteria (nematode dishes containing *E. coli* OP50, i.e., the OP50 group), and the condition with pathogenic *S. aureus* infection but without any intervention material (dishes containing only *S. aureus).* Further groups wherein the intervention substance was added during the culture stage but not during the infection stage, and groups wherein the intervening substance was not added during the culturing stage but was added during the infection stage, were established.

After the nematodes were cultured for a few days, their survival rate was counted on a daily basis. If the nematodes did not respond to a platinum wire, they were considered dead. Two independent assays were carried out for each condition.

Statistical comparative analysis of the survival curves was carried out, and a log rank survival analysis of significance was carried out by using the GraphPad Prism 4 statistics software package. Differences in the effect on nematode survival between groups shown on each day were first analyzed by Two-way ANOVA, and then the comparison of the groups was done by a Tukey's post hoc test. Differences between the group of HMO mix alone or the probiotic alone and the group of the prebiotic-probiotic combination were marked with asterisks in the figures. One-way ANOVA and Dunnett's post hoc test were used to analyze the significant difference in the survival rates of each group on Day 3 or 5 after infection.

### 1.3 Disc diffusion test of inhibition zone

In order to determine whether the test substances *per se* had an antibacterial effect, a disc diffusion test of inhibition zone was carried out. Discs were prepared with sterile filter paper, the test substances HMO (10 mg/mL, 30 mg/mL) or probiotic (1×10⁸ CFU) were introduced thereto, and the discs were dried overnight under sterile conditions. *Staphylococcus aureus* ATCC25923 (1.0×10⁶ CFU/mL) was uniformly applied on the surface of an NGM agar plate, and then the discs were placed on the surface of the inoculated agar plate. Gentamicin (200 µg/mL) was used as a positive control. All plates were incubated at 37°C for 18 hours. Presence or absence of a transparent ring formed around the discs was observed to determine whether the test substances were capable of directly inhibiting the growth of the bacterium or had antibacterial activity.

### 2. Experimental results

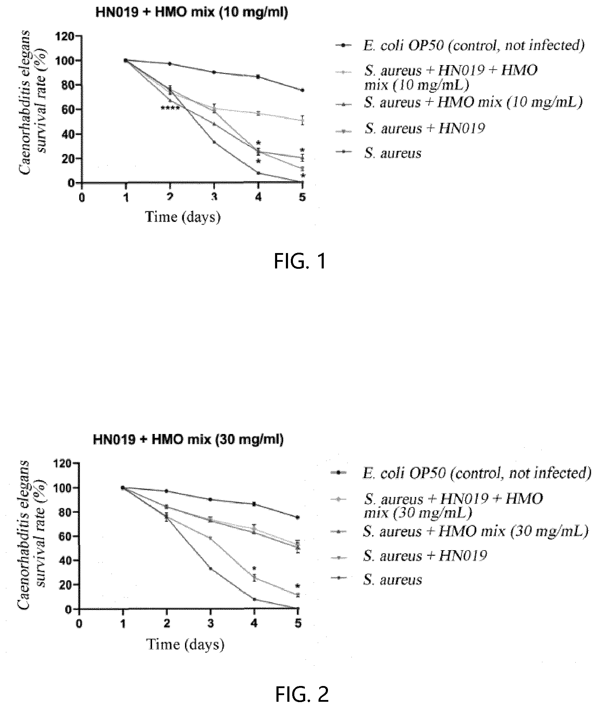

The effects of *Bifidobacterium lactis* HN019 and HMO mix A of five HMOs at two different doses on the survival rate of *C*. *elegans* infected by *Staphylococcus aureus* are shown in Figs. 1 and 2. Throughout the entire experiment course, with the HMO mix at 10 mg/mL, the effect of the prebiotic-probiotic combination was significantly better than that of the HMO mix alone or the probiotic alone (P<0.0001). However, with the HMO mix A at 30 mg/mL, the combination of HMOs and probiotic did not show an advantage throughout the entire experiment course.

As shown in Fig. 1, with the HMO mix A at 10 mg/mL, the effects of the combination of the prebiotic HMOs and the probiotic HN019 on Days 4 and 5 were significantly better than those of the probiotic HN019 alone (p<0.05), and on Days 2, 4 and 5 were significantly better than those of the HMO mix alone (Day 2: p<0.0001; Days 4, 5: p<0.05). Regarding a synergistic effect, the survival rates with the combination of HMOs and probiotic were compared with those with the HMO mix alone or with the probiotic alone, and it was found that the survival rate with the combination of HMOs and probiotic was significantly higher than the sum of the survival rate with the HMO mix alone and the survival rate with the probiotic alone on both Days 4 and 5, indicating a synergistic effect of the combination of HMOs and probiotic (Table 2).

**Table 2**

| Days of experiment | *S*. *aureus* + HMO mix A (10 mg/mL) | *S*. *aureus* + HN019 | *S*. *aureus* + HN019 + HMO mix A (10 mg/mL) | Synergistic effect |
|---|---|---|---|---|
| 1 | 100 | 100 | 100 | No |
| 2 | 67 | 76 | 73 | No |
| 3 | 48 | 58 | 61 | No |
| 4 | 25 | 25 | 56 | Yes |
| 5 | 20 | 11 | 51 | Yes |

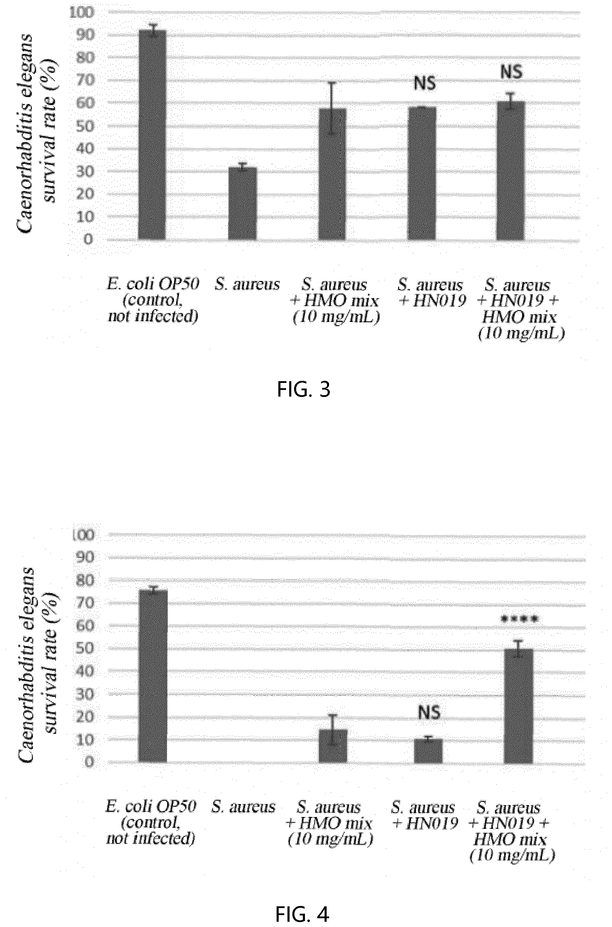

Figs 3 and 4 show a comparison of the effect of the combination of HMO mix A and the probiotic on the survival rate of *Caenorhabditis elegans* on Day 3 and Day 5 of infection. On Day 5 of infection, the combination of the prebiotic and the NH019 showed a better effect than the other test groups.

In addition, further experiments of the present disclosure demonstrate that the survival rate of *Caenorhabditis elegans* in the group with the intervention of the combination of the HMOs and HN019 in the culture stage but without any intervention in the infection stage was significantly higher than that of the groups without intervention either in the culture stage or in the infection stage.

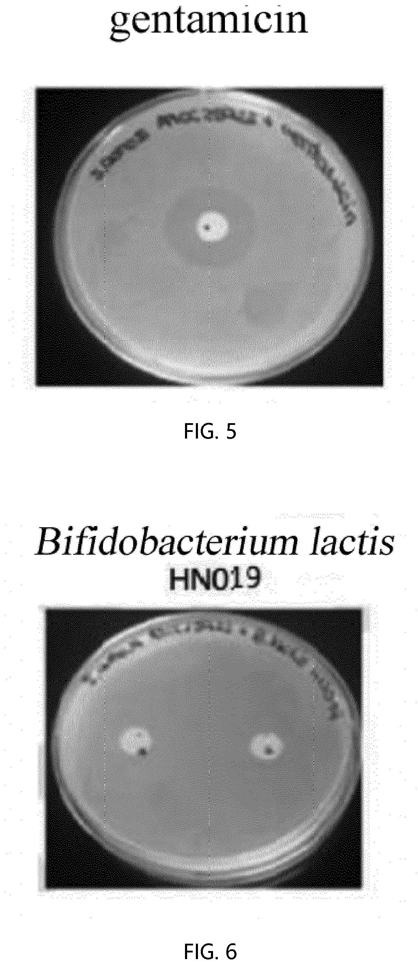

In order to find out whether the protective effect of the combination of HMOs and probiotic against *Staphylococcus aureus* infection shown in the previous tests was due to a direct bactericidal or antibacterial effect, an inhibition zone test was carried out by culturing the test substances together with a bacterial population. As shown in Figs. 5 and 6, the *Bifidobacterium lactis* HN019 strain did not form a transparent inhibition zone around the inoculation site (Fig. 6). The positive control gentamicin showed an antibacterial effect, as indicated by the formation of a transparent inhibition zone (Fig. 5). In addition, the inhibition zone test for each individual HMO at two different concentrations (10 mg/mL, 30 mg/mL) showed a similar result, and none of the individual HMOs could directly inhibit the growth of *S. aureus.* Seen as such, the protective effect of the combination of HMOs and probiotic for *C*. *elegans* against *Staphylococcus aureus* infection shown in the present study was not due to a direct antibacterial action of the substances.

### Example 2: Experiment of efficacy in improving innate immunity and anti-aging with a combination of different HMO mixes and Bifidobacterium lactis HN019

In this example, the components and contents of the tested HMO mixes are shown in Table 3.

**Table 3**

| HMO mix | 2'-FL percentage | 3-FL percentage | LNT percentage | 3'-SL percentage | 6'-SL percentage |
|---|---|---|---|---|---|
| HMO mix A | 53 | 21 | 16 | 5 | 5 |
| HMO mix G | 5 | 41 | 32 | 22 | 0 |
| HMO mix H | 30 | 30 | 25 | 12 | 3 |

Experiment 1 for efficacy of the tested compositions (investigation of the preventive effect of the tested substances in terms of "enhancing resistance of an organism to *Staphylococcus aureus* infection, improving innate immunity of an organism, and/or anti-aging"): nematodes of the same age were taken and cultured in a dish containing a nematode agar medium, and a composition of HMO mix A (10 mg/mL) and the probiotic HN019 (1×10⁸ CFU) in a certain ratio was added for co-culturing until the nematodes grew into an adult form. Then the nematodes were transferred to an infection plate, and were infected by the pathogenic bacterium *Staphylococcus aureus* ATCC25923, without addition of the composition of HMOs and probiotic.

Experiment 2 for efficacy of the tested compositions (investigation of the therapeutic effect of the tested substances in terms of "enhancing resistance of an organism to *Staphylococcus aureus* infection, improving innate immunity of an organism, and/or anti-aging"): nematodes of the same age were taken and cultured in a dish containing a nematode agar medium. When the nematodes grew into an adult form, the nematodes were transferred to a plate supplemented with a composition of HMO mix A (10 mg/mL) and the probiotic HN019 (1×10⁸ CFU) in a certain ratio, and were infected by the pathogenic bacterium *Staphylococcus aureus* ATCC25923.

In addition, another group in which the composition of HMOs and probiotic was added in both the stages before and after the nematodes were infected was compared with the above two cases in Experiments 1 and 2 as a control group.

After the nematodes were cultured for a few days, their survival rate was counted on a daily basis. If the nematodes did not respond to a platinum wire, they were considered dead. Two control groups were further set, one without the pathogenic bacterium (a normal culturing plate supplemented with *E. coli* OP50 as food), and the other one as the infection control group (a plate with only *S. aureus* added). Two independent tests were performed for each condition. Statistical comparative analysis of the survival curves was carried out, and a log rank survival analysis of significance was carried out by using the GraphPad Prism 4 statistics software package.

Other operations during the experiments were done according to Example 1.

### Experimental results

### 1. Effect of test substances co-cultured with nematodes before and/or during infection

As mentioned above, in order to determine whether the test substances have a preventive effect, nematodes were co-cultured with HMO mix A and HN019 only before *S. aureus* infection. In order to determine whether the test substances have a therapeutic effect, nematodes were co-cultured with HMO mix A and HN019 only during *S. aureus* infection. Also included was a case where the test substances were co-cultured with the nematodes both before and during the infection.

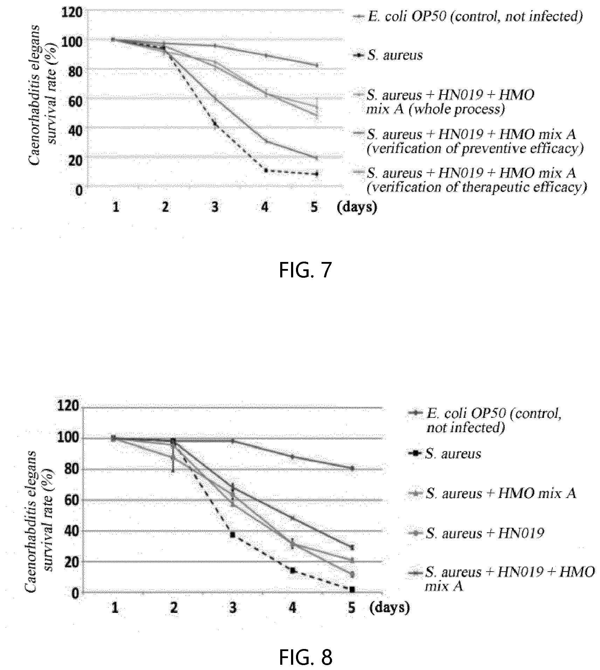

The results are shown in Fig. 7. The survival curve shows that from Day 1 to Day 5 after culturing, no matter whether the test substances were added to the co-culture before the infection or during the infection, there was an improvement in the survival rate and protection for nematodes under an infecting condition with *Staphylococcus aureus.* Both the case where the test substances were added to the nematodes during *Staphylococcus aureus* infection, and the case where the test substances were present in the whole process, had similar effects in terms of the improvement in survival rate and protection against *Staphylococcus aureus.* Furthermore, as compared with the case where the test substances were added only before the infection, stronger protection for the survival of nematodes was seen in both the case where the test substances were added during the infection and the case where the test substances were present in the whole process.

### 2. Effect of HMO mix A (8 mg/mL) and Bifidobacterium lactis HN019, alone or in combination, on the survival rate of nematodes under Staphylococcus aureus infection

The results are shown in Fig. 8. It can be seen that from Day 1 to Day 5 after culturing, both the HMO mix A alone at 8 mg/mL and the probiotic HN019 (10⁸ cfu) alone can significantly improve the survival rate of nematodes under an infecting condition with *Staphylococcus aureus,* and when they were combined, the improvement was even more significant (P<0.0001). This suggests there is an excellent synergistic effect between the probiotic HN019 and HMO mix A (8 mg/mL).

### 3. Effect of HMO mix G (8 mg/mL) and Bifidobacterium lactis HN019, alone or in combination, on the survival rate of nematodes under Staphylococcus aureus infection

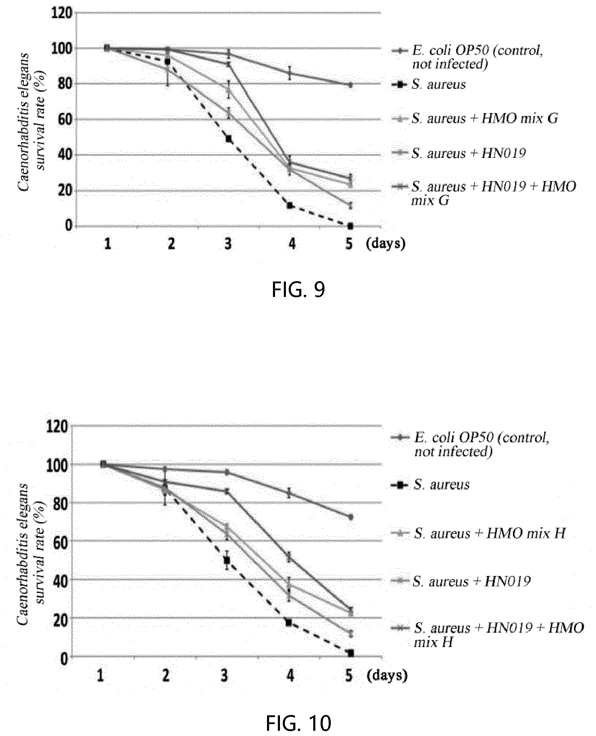

The results are shown in Fig. 9. It can be seen that from Day 1 to Day 5 after culturing, both HMO mix G alone at 8 mg/mL and the probiotic HN019 (10⁸ cfu) alone can significantly improve the survival rate of nematodes under an infecting condition with *Staphylococcus aureus,* and when they were combined, the improvement was even more significant. This suggests there is an excellent combinational effect between the probiotic HN019 and HMO mix G (8 mg/mL).

### 4. Effect of HMO mix H (8 mg/mL) and Bifidobacterium lactis HN019, alone or in combination, on the survival rate of nematodes under Staphylococcus aureus infection

The results are shown in Fig. 10. It can be seen that from Day 1 to Day 5 after culturing, both HMO mix H alone at 8 mg/mL and the probiotic HN019 (10⁸ cfu) alone can significantly improve the survival rate of nematodes under an infecting condition with *Staphylococcus aureus,* and when they were combined, the improvement was even more significant. This suggests there is an excellent combinational effect between the probiotic HN019 and HMO mix H (8 mg/mL).

### Example 3: Infant formula powder with addition of a composition of a HMO mix and Bifidobacterium lactis HN019

This example provided formula powder for infant 0-6 months old. In this formula powder, the total protein content was 11.1g/100 g powder, the fat content was 28.3 g/100 g powder, the carbohydrate content was 52.9 g/100 g powder, the content of the HMO mix was 2.5 g/100 g powder, and the *Bifidobacterium lactis* was 4×10⁸ CFU/100 g. The components and their ratio in the HMO mix were: 2'-fucosyllactose (2'-FL) : 3'-fucosyllactose (3'-FL) : lactose-N-tetraose (LNT) : 3'-sialyllactose (3'-SL) : 6'-sialyllactose (6'-SL) = 53 : 21 : 16 : 5 : 5.

## Claims

1. A composition comprising *Bifidobacterium lactis* and human milk oligosaccharides (HMOs).

2. The composition according to claim 1, wherein the *Bifidobacterium lactis* includes the strain *Bifidobacterium lactis* HN019.

3. The composition according to claim 1 or 2, wherein the HMOs include one or more of 2'-fucosyllactose, 3'-fucosyllactose, lactose-N-tetraose, 3'-sialyllactose, and 6'-sialyllactose;
preferably, the HMOs include at least three or four of 2'-fucosyllactose, 3'-fucosyllactose, lactose-N-tetraose, 3'-sialyllactose, and 6'-sialyllactose in a weight ratio of (0% to 73%) : (0% to 61%) : (0% to 52%) : (0% to 42%) : (0% to 25%);
more preferably, the HMOs include 2'-fucosyllactose, 3'-fucosyllactose, lactose-N-tetraose, 3'-sialyllactose, and 6'-sialyllactose in a weight ratio of (0% to 53%) : (21% to 41%): (16% to 32%): (5% to 22%) : (0% to 5%).

4. The composition according to claim 1 or 2, wherein the ratio of the *Bifidobacterium lactis* to the HMOs is (1×10³ to 1×10¹² CFU) : (0.1 to 10 g), preferably (1×10⁶ to 1×10¹⁰CFU): (1 to 10 g).

5. Use of the composition according to any one of claims 1 to 4 in the manufacture of a food product or medicament for effectively enhancing resistance of an organism to *Staphylococcus aureus* infection, improving innate immunity of an organism, and/or anti-aging.

6. The use according to claim 5, wherein the enhancing resistance of an organism to *Staphylococcus aureus* infection includes: enhancing an individual's ability to prevent *Staphylococcus aureus* infection, reducing the ability of *Staphylococcus aureus* to infect an individual, and/or alleviating food poisoning, enteritis, pneumonia, skin infection, wound ulceration, and/or meningitis caused by *Staphylococcus aureus* infection in an individual.

7. The use according to claim 5, wherein the food product is a liquid beverage, a solid beverage, an oral liquid, a diary product, a tablet, or a capsule, such as milk powder, particularly infant formula powder.

8. The use according to claim 5 or 7, wherein the *Bifidobacterium lactis* in the composition is used in an amount of 1×10³ to 1×10¹² CFU per day, preferably 1×10⁶ to 1×10¹¹ CFU per day.

9. The use according to claim 5 or 7, wherein the HMOs in the composition are used in an amount of 0.015 to 15 g per day, further preferably 0.1 to 12 g per day, and more preferably 1.5 to 10 g per day.

10. A food product comprising the composition according to any one of claims 1 to 4;
preferably, the *Bifidobacterium lactis* in the food product is used in an amount of 1×10³ to 1×10¹² CFU per day, and more preferably 1×10⁶ to 1×10¹¹ CFU per day;
preferably, the HMOs in the food product are used in an amount of 0.015 to 15 g per day, further preferably 0.1 to 12 g per day, and more preferably 1.5 to 10 g per day.

11. Use of HMOs in improving the potency of *Bifidobacterium lactis* HN019 in enhancing resistance of an organism to *Staphylococcus aureus* infection, improving innate immunity of an organism, and/or anti-aging.

12. A method for enhancing resistance of an organism to *Staphylococcus aureus* infection, improving innate immunity of an organism, and/or anti-aging, comprising:
administering to the organism an effective amount of the composition according to any one of claims 1 to 4.

13. The method according to claim 12, wherein the enhancing resistance of an organism to *Staphylococcus aureus* infection includes:
enhancing an individual's ability to prevent *Staphylococcus aureus* infection,
reducing the ability of *Staphylococcus aureus* to infect an individual, and/or
alleviating food poisoning, enteritis, pneumonia, skin infection, wound ulceration, and/or meningitis caused by *Staphylococcus aureus* infection in an individual.

14. The method according to claim 12 or 13, wherein the composition according to any one of claims 1 to 4 is administered to the organism in the form of a food product, wherein the food product is a liquid beverage, a solid beverage, an oral liquid, a diary product, a tablet, or a capsule, such as milk powder, particularly infant formula powder;
preferably, the *Bifidobacterium lactis* in the food product is used in an amount of 1×10³ to 1×10¹² CFU per day, and more preferably 1×10⁶ to 1×10¹¹ CFU per day;
preferably, the HMOs in the food product are used in an amount of 0.015 to 15 g per day, further preferably 0.1 to 12 g per day, and more preferably 1.5 to 10 g per day.
